# EUROPEAN PATENT APPLICATION

(11) **EP 3 103 860 A1**
(43) Date of publication of application: **14.12.2016**
(21) Application number: 15746071.8
(22) Date of filing: 06.02.2015
(51) Int. Cl.: C12M 1/00, C12M 1/02, C12M 1/04, C12M 3/00

(54) **CULTURING DEVICE**

(30) Priority: 07.02.2014 JP 2014022543
(71) Applicant: Tokyo Electron Limited, Tokyo 107-6325 (JP)
(72) Inventor: MORI Kiyoshi, Tokyo 107-6325 (JP)
(74) Representative: Diehl & Partner GbR
(86) International application number: PCT/JP2015/053420
(87) International publication number: WO 2015/119254

(57) **Abstract**

A culturing device (11, 20, 30) is provided with an incubator unit (100). The incubator unit (100) comprises: an internal housing (101); a placement unit (120) that is installed in the internal housing (101) and is for placing a container (75) containing cells; a plurality of heating units (140) that are installed in the internal housing (101) and are installed so as to be symmetrical with respect to a symmetrical axis that extends in the vertical direction; an air circulation unit (150) that is installed in a center part at an upper position in the internal housing (101), suctions the air inside of the internal housing (101), and exhausts the suctioned air; and a dispersion member (160) that disperses the air, which was exhausted from the air circulation unit (150), inside the internal housing (101) so that the air disperses symmetrically with respect to the symmetrical axis.

## Description

### TECHNICAL FIELD

The present disclosure relates to a culturing device configured to culture cells.

### BACKGROUND

In the related art, there is known a culturing device for use in culturing cells. In this culturing device, an incubator for keeping a temperature or the like at a constant level is used. One example of the incubator is disclosed in, for example, Japanese laid-open publication No. 2000-37184. Japanese laid-open publication No. 2000-37184 discloses an incubator which includes a housing, an environment condition adjustment means for adjusting environment conditions within the housing, a mounting parts installed within the housing and configured to mount a container which stores a sample, and a processing means configured to perform a sterilizing or cleaning process with respect to the interior of the housing.

Another example of the incubator is disclosed in Japanese laid-open publication No. 2000-93156. Japanese laid-open publication No. 2000-93156 discloses an incubator which includes a housing, a sample table disposed within the housing and configured to mount a plurality of containers which stores samples, an environment control part configured to keep an internal environment of the housing under predetermined conditions, a first opening portion formed in the housing, a first openable/closable door configured to close the first opening portion, a second opening portion which is smaller than the first opening portion formed in the housing or the first door and which has a size capable of allowing at least one container to pass therethrough, and a second openable/closable door configured to close the second opening portion.

### SUMMARY

However, the incubators provided in the related art fail to sufficiently make uniform a temperature or the like within the housing.

The present disclosure provides a culturing device provided with an incubator part capable of making uniform, with high accuracy, a temperature, a gas distribution or the like within a housing.

According to one embodiment of the present invention, there is provided a culturing device, including:
an incubator part,
wherein the incubator part includes:
   an internal housing;
   a mounting part installed within the internal housing and configured to mount thereon a container which stores cells;
   a plurality of heating parts installed within the internal housing and disposed in a symmetrical relationship with respect to an axis of rotational symmetry extending in an up-down direction;
   a gas circulation part installed in an upper central position within the internal housing and configured to draw a gas existing within the internal housing and to discharge the gas thus drawn; and
   a diffusion member configured to diffuse the gas discharged from the gas circulation part within the internal housing in a symmetrical relationship with respect to the axis of rotational symmetry.

In the culturing device according to the present disclosure, the diffusion member may be configured to diffuse the gas discharged from the gas circulation part within the internal housing so that the gas is blown toward each of the heating parts or an upper side of each of the heating parts.

In the culturing device according to the present disclosure, the incubator part may further include: a heat insulating member installed between the mounting part and the heating parts so as to surround at least a portion of a peripheral edge of the mounting part, and
a gap may be formed at a lower side of the heat insulating member between the heat insulating member and a member adjoining the heat insulating member.

In the culturing device according to the present disclosure, the incubator part may further include: a drive part configured to rotate the mounting part about the axis of rotational symmetry

In the culturing device according to the present disclosure, the incubator part may further include: an adjustment gas supply part configured to supply an adjustment gas to the gas circulation part, and
the adjustment gas supply part may be capable of adjusting a concentration of the adjustment gas in the gas existing within the internal housing.

The culturing device according to the present disclosure may further include:
an adjustment gas sterilization part configured to sterilize the adjustment gas supplied from the adjustment gas supply part.

In the culturing device according to the present disclosure, the adjustment gas may include oxygen and the adjustment gas sterilization part may be configured to irradiate ultraviolet rays on the adjustment gas.

In the culturing device according to the present disclosure, the incubator part may further include: a humidification water storage part disposed under the mounting part and configured to store humidification water.

In the culturing device according to the present disclosure, the incubator part may further include: a detection part configured to detect whether the container is mounted on the mounting part.

In the culturing device according to the present disclosure, the mounting part may include a plurality of mounting parts, and
the internal housing may include opening/closing doors installed in a corresponding relationship with the respective mounting parts.

In the culturing device according to the present disclosure, the number of the mounting parts may be equal to the number of the opening/closing doors.

In the culturing device according to the present disclosure, the incubator part may further include: a holding part extending in the up-down direction along the axis of rotational symmetry and configured to hold the mounting part in a horizontal direction, and
the mounting part may include a first mounting portion held by the holding part, a second mounting portion positioned outside a peripheral edge of the first mounting portion and held by the first mounting portion, and an elastic member disposed between the first mounting portion and the second mounting portion.

In the culturing device according to the present disclosure, the mounting part may include a plurality of mounting parts, and
each of the mounting parts may be mounted above an adjoining lower mounting part so that each of the mounting parts can be moved in the up-down direction with respect to the adjoining lower mounting part.

The culturing device according to the present disclosure may further include:
an external housing disposed so as to surround the internal housing;
an external gas supply part installed at an upper side within the external housing and configured to supply a gas into the external housing; and
an external gas discharge part installed at a lower side within the external housing and configured to discharge the gas supplied from the external gas supply part.

The culturing device according to the present disclosure may further include:
an external housing disposed so as to surround the internal housing; and
a sterilization part installed within the external housing and configured to sterilize the gas supplied from the supply part.

In the culturing device according to the present disclosure, the sterilization part may be a sterilization lamp, and
a light shielding plate configured to prevent sterilization light irradiated from the sterilization lamp from leaking out of the external housing may be installed below the sterilization lamp.

In the culturing device according to the present disclosure, the sterilization part may be a sterilization lamp,
at least a portion of the external housing may be transparent or translucent, and
a transparent or translucent portion of the external housing may be made of a material which prevents sterilization light irradiated from the sterilization lamp from leaking out of the external housing.

The culturing device according to the present disclosure may further include:
a delivery housing disposed adjacent to the internal housing;
a transfer mechanism installed within the delivery housing and configured to transfer the container into and out of the internal housing; and
an up/down drive mechanism configured to move the transfer mechanism in the up-down direction.

In the culturing device according to the present disclosure, the mounting part may include a disc-shaped plate in which a plurality of arc-shaped slits is formed in a concentric relationship, and
the arc-shaped slits adjoining in the circumferential direction may be closed in an expected container mounting position to form a closed portion.

In the culturing device according to the present disclosure, a distance of the closed portion in the circumferential direction may be substantially equal to a distance of the container in the circumferential direction.

### BRIEF DESCRIPTION OF THE DRAWINGS

**FIG. 1** is a schematic top plane view illustrating a configuration of an automatic culture system according to an embodiment of the present disclosure.
**FIG. 2** is a control block diagram of the automatic culture system according to the embodiment of the present disclosure.
**FIG. 3** is a front view illustrating a container transfer part employed in the embodiment of the present disclosure.
**FIG. 4** is a perspective view illustrating an external housing and so forth, which surround an incubator part employed in the embodiment of the present disclosure.
**FIG. 5** is a perspective view illustrating a gas flow within the external housing employed in the embodiment of the present disclosure.
**FIG. 6** is a perspective view illustrating a sterilization lamp, a light shielding plate and the like, which are installed within the external housing employed in the embodiment of the present disclosure.
**FIG. 7** is a perspective view illustrating the incubator part employed in the embodiment of the present disclosure.
**FIG. 8** is a perspective view illustrating a state in which thermal insulation doors of the incubator part employed in the embodiment of the present disclosure are removed.
**FIG. 9** is a perspective view illustrating a state in which a heat insulation member and a humidification water tray are removed from the state illustrated in **FIG. 8****.**
**FIG. 10** is a side sectional view illustrating an internal structure of an internal housing employed in the embodiment of the present disclosure.
**FIG. 11A** is a top plane view of a mounting part employed in the embodiment of the present disclosure, which is viewed from above.
**FIG. 11B** is a top plane view of a modification of the mounting part employed in the embodiment of the present disclosure, which is viewed from above.
**FIG. 12** is a side sectional view of the mounting part and the like employed in the embodiment of the present disclosure.
**FIG. 13** is a perspective view illustrating a detection part and the like employed in the embodiment of the present disclosure.
**FIG. 14** is a control block diagram of members associated with the incubator part according to the embodiment of the present disclosure.
**FIG. 15** is a side sectional view illustrating an example in which a transfer housing is disposed adjacent to the internal housing of the incubator part.
**FIG. 16** is a graph showing the measurement results of temperature pull-up characteristics according to an example of the present disclosure and comparative examples.
**FIG. 17** is a graph showing the measurement results of temperature drop characteristics at the time of power outage, in an example of the present disclosure and comparative examples.
**FIG. 18** is a graph showing the measurement results of temperature return characteristics at the time of door opening, in an example of the present disclosure and comparative examples.
**FIG. 19** is a graph showing the measurement results of carbon dioxide concentration return characteristics at the time of door opening, in an example of the present disclosure and comparative examples.

### DETAILED DESCRIPTION

### Embodiment

### «Configuration»

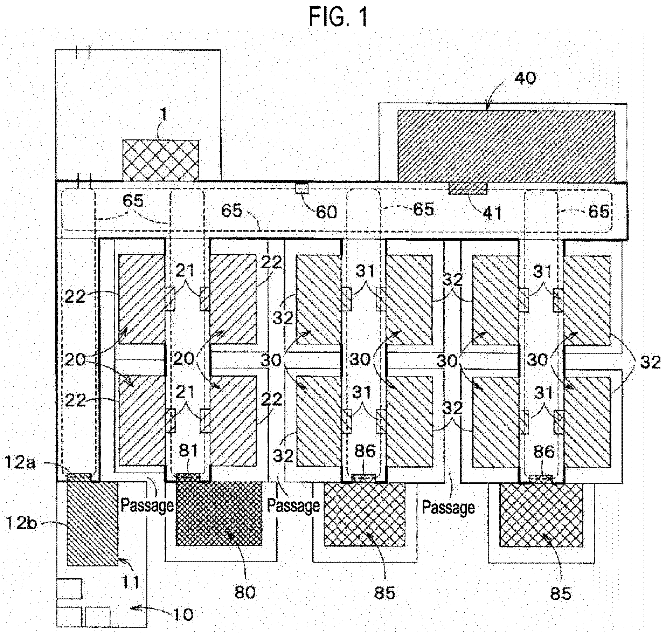

An embodiment of a culturing device according to the present disclosure will now be described with reference to the accompanying drawings. **FIGS. 1** to **19** are views for explaining the embodiment of the present disclosure.

The culturing device of this embodiment can be used to culture all kinds of cells and can be used when culturing different cells including pluripotent stem cells such as (human) iPS cells, (human) ES cells or the like, chondrocytes such as bone marrow stromal cells (MSC) or the like, dendritic cells, and so forth. In this embodiment, descriptions will be made hereinafter using an automatic culture system for automatically culturing iPS cells. However, it should be noted that this is nothing more than one example. In other words, it should be noted that the culturing device according to the present disclosure can be used in a case where cells are not automatically cultured.

### [Overall Configuration]

First, a device configuration of an automatic culture system according to this embodiment will be described.

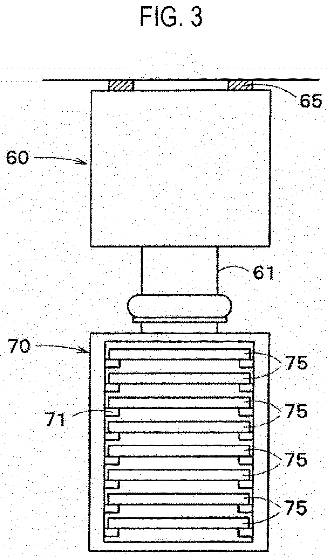

As illustrated in **FIG. 1****,** the automatic culture system of this embodiment includes a raw material storage device 10 configured to store raw material cells, a container transfer part 60 configured to transfer a first airtight container 70 *(see* **FIG. 3**) which accommodates cells in a sealed state, and automatic culture devices 20 and 30 configured to receive the first airtight container 70 transferred by the container transfer part 60, configured to take out second airtight containers 75 (corresponding to a "container" recited in the claims), which accommodate cells, from the first airtight container 70 and configured to culture therein the cells contained in the taken-out second airtight containers 75. In this embodiment, descriptions will be made hereinafter mainly based on a premise that the "container" recited in the claims is the second airtight container 75. However, the present disclosure is not limited to this aspect. It should be noted that different containers such as a culture-purpose dish, a culture-purpose petri dish and the like can be used.

In this embodiment, as mentioned above, an aspect using iPS cells will be described. Thus, the raw material storage device 10 includes an iPS cell establishing device 11 which establishes iPS cells. In addition, the raw material storage device 10 includes a unit thermostatic bath, a centrifuge, an automatic blood cell counting device, an automatic magnetic cell separator, a flow cytometer, a gene introduction device, and so forth.

The automatic culture devices 20 and 30 of this embodiment include a plurality of (four, in the aspect illustrated in **FIG. 1**) iPS cell automatic culture devices 20 which automatically culture iPS cells and a plurality of (eight, in the aspect illustrated in **FIG. 1**) differentiated cell automatic culture devices 30 which automatically culture differentiated cells differentiated from the iPS cells. In this embodiment, when merely saying "automatic culture devices", they refer to the iPS cell automatic culture devices 20, the differentiated cell automatic culture devices 30, or both of the iPS cell automatic culture devices 20 and the differentiated cell automatic culture devices 30. Unless otherwise specified, when merely saying "cells" in this embodiment, they refer to raw material cells such as body cells or the like serving as a source of iPS cells, iPS cells, differentiated cells, or two or all of the raw material cells, the iPS cells and the differentiated cells.

As illustrated in **FIG. 3****,** the first airtight container 70 includes a plurality of (eight, in **FIG. 3**) racks 71 for mounting the respective second airtight containers 75 thereon. The second airtight containers 75 are mounted on the respective racks 71. In a state in which the second airtight containers 75 are accommodated within the first airtight container 70, the first airtight container 70 is transferred by the container transfer part 60. The second airtight containers 75 may accommodate not only the cells but also materials such as a liquid culture medium, a chemical and the like, which will be described later.

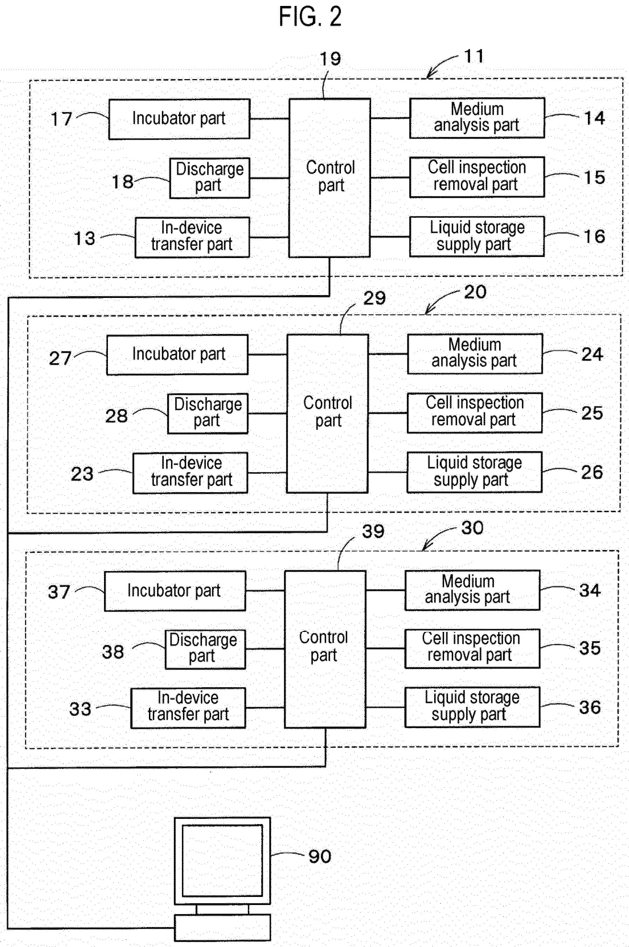

The iPS cell automatic culture device 20 includes: a housing 22 illustrated in **FIG. 1****;** a medium analysis part 24 illustrated in **FIG. 2****,** which analyzes liquid culture medium components that vary with the culture of iPS cells; a cell inspection removal part 25 which inspects the iPS cells and performs removal of the iPS cells having a bad state; a liquid storage supply part 26 which stores and supplies a liquid including a liquid culture medium or a proteolytic enzyme and which performs a pre-treatment before iPS cells are seeded, seeds iPS cells or recovers iPS cells; an incubator part 27 which holds the second airtight container 75 and automatically adjusts one or all of a temperature, a humidity and a gas concentration; and a discharge part 28 for discharging downward from the housing 22 a waste liquid including a used liquid culture medium, a used cleaning liquid, a used reagent or the like, which is used within the iPS cell automatic culture device 20. Furthermore, the iPS cell automatic culture device 20 includes an in-device transfer part 23 which transfers the second airtight container 75 and the like within the iPS cell automatic culture device 20. The liquid storage supply part 26 described above has a function of inverting the second airtight container 75 upside down. Incidentally, in the case where the second airtight container 75 is used as in this embodiment, the humidity within the incubator part 27 may not be particularly managed. It is therefore possible to simplify the management of a cell culture environment. By employing the second airtight container 75 of this type, there is no fear that contamination from the air occurs. Furthermore, the transfer becomes easy.

The liquid storage supply part 26 described above appropriately supplies a liquid culture medium from an inlet (not shown) into the second airtight container 75, thereby automatically remounting position an old liquid culture medium existing within the second airtight container 75 with a new liquid culture medium. Based on the information of the iPS cells acquired, the cell inspection removal part 25 selectively peels off defective iPS cells from an ECM (Extracellular Matrix) coated on a surface of a film (not shown) of the second airtight container 75. Thereafter, the liquid storage supply part 26 supplies a liquid culture medium from the inlet into the second airtight container 75, thereby pushing out floating defective iPS cells from the second airtight container 75 through an outlet (not shown). As the method of selectively peeling off the iPS cells existing within the second airtight container 75, use a method of irradiating ultrasonic waves or light on the iPS cells or a method of applying a physical force from the outside of the second airtight container 75, may be used. When using this method, a proteolytic enzyme may be used in combination.

Furthermore, the liquid storage supply part 26 appropriately supplies a proteolytic enzyme from the inlet into the second airtight container 75, thereby peeling off the iPS cells from the ECM coated on a surface of a film 77 of the second airtight container 75. Thereafter, the liquid storage supply part 26 supplies a liquid culture medium from the inlet into the second airtight container 75, whereby floating iPS cells are pushed out from the second airtight container 75 through the outlet. The iPS cells thus pushed out are diluted into a suspension and are then accommodated (seeded) within a plurality of other second airtight containers 75. In this way, the iPS cell automatic culture device 20 automatically performs the subculture of the iPS cells.

The internal temperature of the iPS cell automatic culture device 20 is adjusted by the incubator part 27 so that the internal temperature becomes, for example, about 37 degrees C. Furthermore, the gas concentration within the iPS cell automatic culture device 20 is adjusted by the incubator part 27 by appropriately adding carbon dioxide or nitrogen. If necessary, the humidity may be adjusted by the incubator part 37 so as to become about 100%.

The differentiated cell automatic culture device 30 includes: a housing 32 illustrated in FIG. 1; a medium analysis part 34 illustrated in **FIG. 2****,** which analyzes liquid culture medium components that vary with the culture of differentiated cells; a cell inspection removal part 35 which inspects the differentiated cells and performs removal of the differentiated cells having a bad state; a liquid storage supply part 36 which stores and supplies a liquid including a liquid culture medium or a proteolytic enzyme and which performs a pre-treatment before differentiated cells are seeded, seeds differentiated cells or recovers differentiated cells; an incubator part 37 which holds the second airtight container 75 and automatically adjusts one or all of a temperature, a humidity and a gas concentration; and a discharge part 38 for discharging downward from the housing 32 a waste liquid including a used liquid culture medium, a used cleaning liquid, a used reagent or the like, which is used within the differentiated cell automatic culture device 30. Furthermore, the differentiated cell automatic culture device 30 includes an in-device transfer part 33 which transfers the second airtight containers 75 or the like within the differentiated cell automatic culture device 30.

The liquid storage supply part 36 described above appropriately supplies a liquid culture medium from the inlet into the second airtight container 75, thereby automatically remounting the position of an old liquid culture medium existing within the second airtight container 75 with a new liquid culture medium. Based on the information of the differentiated cells acquired, the cell inspection removal part 35 selectively peels off defective differentiated cells from the ECM coated on the surface of the film 77 of the second airtight container 75. Thereafter, the liquid storage supply part 36 supplies a liquid culture medium from the inlet into the second airtight container 75, thereby pushing out the floating defective differentiated cells from the second airtight container 75 through the outlet. As the method of selectively peeling off the differentiated cells existing within the second airtight container 75, a method of irradiating ultrasonic waves or light on the differentiated cells or a method of applying a physical force from the outside of the second airtight container 75, may be used. When using this method, a proteolytic enzyme may be used in combination.

Furthermore, the liquid storage supply part 36 appropriately supplies a proteolytic enzyme from the inlet into the second airtight container 75, thereby peeling off the differentiated cells from the ECM coated on the surface of the film 77 of the second airtight container 75. Thereafter, the liquid storage supply part 36 supplies a liquid culture medium from the inlet into the second airtight container 75, whereby the floating differentiated cells are pushed out from the second airtight container 75 through the outlet. The differentiated cells thus pushed out are diluted into a suspension and are then accommodated (seeded) within a plurality of other second airtight containers 75. In this way, the differentiated cell automatic culture device 30 automatically performs the subculture of the differentiated cells.

The internal temperature of the differentiated cell automatic culture device 30 is adjusted by the incubator part 37 so that the internal temperature becomes, for example, about 37 degrees C. Furthermore, the gas concentration within the differentiated cell automatic culture device 30 is adjusted by the incubator part 37 by appropriately adding carbon dioxide or nitrogen. When inducing differentiation, the liquid storage supply part 36 of the differentiated cell automatic culture device 30 may supply a liquid culture medium including a differentiation-inducing factor. If necessary, the humidity may be adjusted by the incubator part 37 so as to become about 100%.

As illustrated in **FIG. 2****,** the iPS cell automatic culture device 20 includes a control part 29 which is connected to each of the medium analysis part 24, the cell inspection removal part 25, the liquid storage supply part 26, the incubator part 27, the discharge part 28 and the in-device transfer part 23 so as to make communication therewith and which is configured to control them. The control part 29 has a function of, with respect to the iPS cell automatic culture device 20, managing the status, managing the log, managing the culture schedule, or serving as a user interface. Furthermore, the differentiated cell automatic culture device 30 includes a control part 39 which is connected to each of the medium analysis part 34, the cell inspection removal part 35, the liquid storage supply part 36, the incubator part 37, the discharge part 38 and the in-device transfer part 33 so as to make communication therewith and which is configured to control them. The control part 29 has a function of, with respect to the differentiated cell automatic culture devices 30, managing the status, managing the log, managing the culture schedule, or serving as a user interface.

The iPS cell establishing device 11 is similar in configuration to the iPS cell automatic culture device 20 and the differentiated cell automatic culture device 30. That is to say, the iPS cell establishing device 11 includes a housing 12b illustrated in **FIG. 1****;** a medium analysis part 14 illustrated in **FIG. 2****,** which analyzes a liquid culture medium; a cell inspection removal part 15 which inspects the raw material cells and performs removal of the raw material cells having a bad state; a liquid storage supply part 16 which stores and supplies a liquid including a liquid culture medium or a proteolytic enzyme; an incubator part 17 which automatically adjusts one or all of a temperature, a humidity and a gas concentration within the housing 12b; and a discharge part 18 for discharging downward from the housing 12b a waste liquid including a used liquid culture medium, a used cleaning liquid, a used reagent or the like, which is used within the iPS cell establishing device 11. Furthermore, the iPS cell establishing device 11 includes an in-device transfer part 13 which transfers the second airtight container 75 within the iPS cell establishing device 11. Moreover, the iPS cell establishing device 11 includes a control part 19 which is connected to each of the medium analysis part 14, the cell inspection removal part 15, the liquid storage supply part 16, the incubator part 17, the discharge part 18 and the in-device transfer part 13 so as to make communication therewith and which is configured to control them. The respective control parts 19, 29 and 39 are connected to an external device 90 such as, e.g., a personal computer or the like.

As illustrated in **FIG. 3****,** the container transfer part 60 of this embodiment includes a central holding part 61 which holds the first airtight container 70 such that the first airtight container 70 is suspended downward. The container transfer part 60 is configured to move along a rail 65 provided in a ceiling.

As illustrated in **FIG. 1****,** the iPS cell automatic culture device 20 includes a loading part 21 for loading the second airtight container 75 from the first airtight container 70 therethrough. The loading part 21 may include a cell loading/unloading part (not shown) for loading the iPS cells accommodated in the second airtight container 75 therethrough and for unloading the cultured iPS cells therefrom, and a material loading part (not shown) for loading materials accommodated in the second airtight container 75 therethrough. Similarly, as illustrated in **FIG. 1****,** the differentiated cell automatic culture device 30 includes a loading part 31 configured to load the second airtight container 75 from the first airtight container 70 therethrough. The loading part 31 may include a cell loading/unloading part (not shown) for loading the iPS cells accommodated in the second airtight container 75 therethrough and for unloading the cultured differentiated cells therefrom, and a material loading part (not shown) for loading materials accommodated in the second airtight container 75 therethrough. In this embodiment, the materials include a liquid culture medium, a reagent, a cleaning liquid, a culture plate, a vial, a filter, a needle, and so forth. Furthermore, as illustrated in **FIG. 1****,** the iPS cell establishing device 11 includes a loading part 12a configured to load the first airtight container 70 therethrough.

As illustrated in **FIG. 1****,** the automatic culture system of this embodiment includes: a sterilizing device 1 for sterilizing the interior of the first airtight container 70; an iPS cell analysis device 80 which receives the iPS cells cultured in the iPS cell automatic culture device 20 from the loading part 81 at a predetermined timing and which inspects the iPS cells thus received; a differentiated cell analysis device 85 which receives the differentiated cells cultured in the differentiated cell automatic culture devices 30 from the loading part 86 at a predetermined timing and which inspects the differentiated cells thus received; and a freezing storage device 40 which receives the iPS cells, the differentiated cells or both cultured in the automatic culture devices 20 and 30 from a loading part 41 and which freezes and stores the iPS cells, the differentiated cells or both thus received. There may be provided multiple freezing storage devices 40. The entire room may be cooled and may serve as a freezer. In the case where multiple freezing storage devices 40 are provided in the room or in the case where the room itself serves as a freezer, a rail 65 may be provided in the ceiling of the room so that the container transfer part 60 can move along the rail 65.

One example of the sterilizing device 1 described above may include a sterilizing device which sterilizes the interior of the first airtight container 70 by supplying a sterilizing gas such as a hydrogen peroxide gas or a high-temperature gas into the first airtight container 70. Another example of the sterilizing device 1 may include a sterilizing device which sterilizes the interior of the first airtight container 70 by irradiating, for example, γ rays or ultraviolet rays from the outside while keeping the first airtight container 70 in a sealed state. In some embodiments, before the first airtight container 70 is loaded from the outside, the interior of the first airtight container 70 may be sterilized using, for example, γ rays or ultraviolet rays. There may be a case where the liquid culture medium or the like contains protein or the like which is broken by γ rays or ultraviolet rays. In this case, it is desirable that sterilization is performed by a sterilizing gas such as a hydrogen peroxide gas, a high-temperature gas or the like.

### «Configuration around Incubator Part»

Next, a configuration around the aforementioned incubator part will be described. In the following descriptions, one, two or all of the incubator part 17 of the iPS cell establishing device 11, the incubator part 27 of the iPS cell automatic culture device 20 and the incubator part 37 of the differentiated cell automatic culture device 30 will be referred to as an "incubator part 100". In this embodiment, the incubator part 17 of the iPS cell establishing device 11, the incubator part 27 of the iPS cell automatic culture device 20 and the incubator part 37 of the differentiated cell automatic culture device 30 have the same configuration.

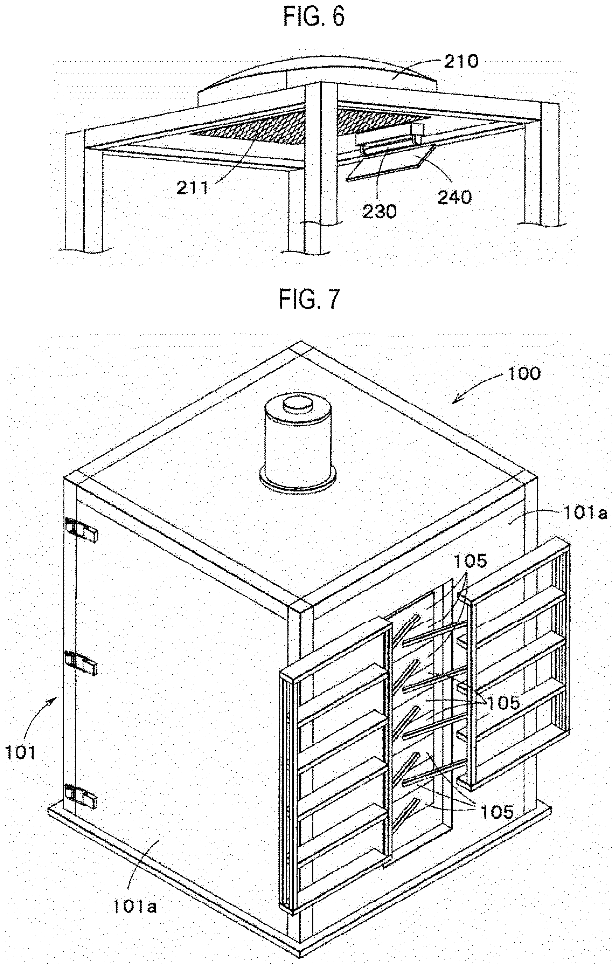

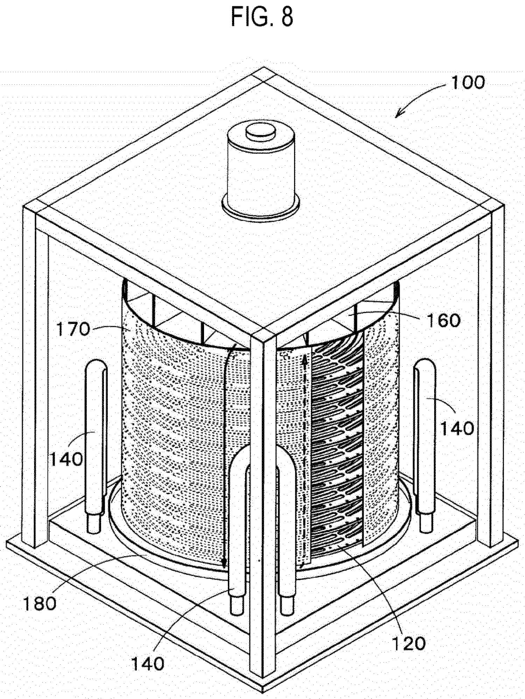

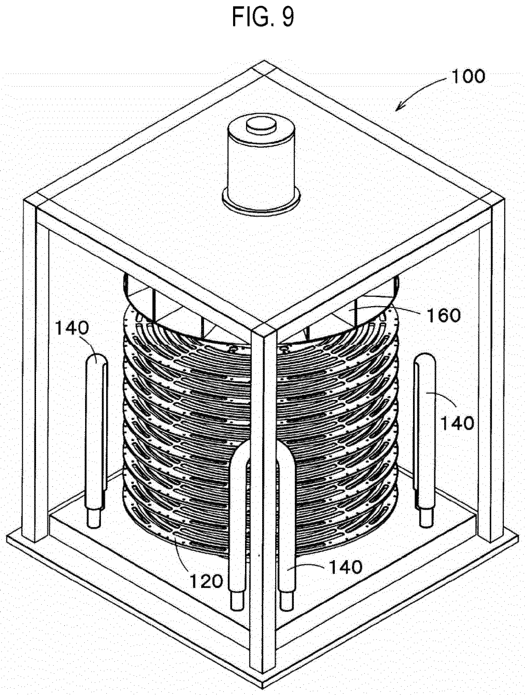

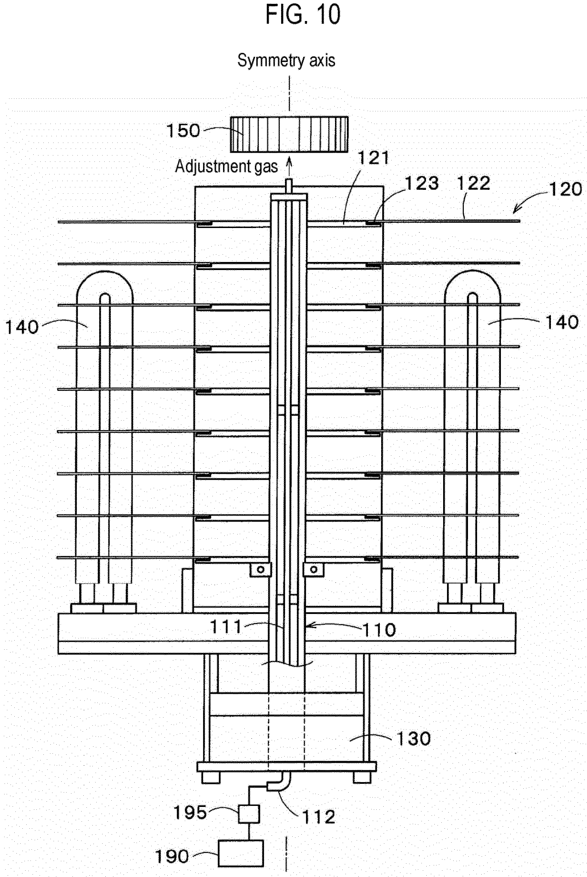

The incubator part 100 includes an internal housing 101 illustrated in **FIG. 7****.** As illustrated in **FIG. 10****,** the incubator part 100 includes: a central shaft 110 (corresponding to a "central holding part" of the claims) installed within the internal housing 101 to extend along an axis of rotational symmetry extending in an up-down direction; a plurality of (nine, in this embodiment) mounting parts 120 horizontally held by the central shaft 110 and configured to mount thereon the second airtight container 75 which accommodates cells; a drive part 130 such as a motor or the like configured to rotate the respective mounting parts 120 by rotating the central shaft 110; and a plurality of heating parts 140 installed within the internal housing 101. As illustrated in **FIGS. 8** and **9****,** the heating parts 140 are installed in a symmetrical relationship with respect to the axis of rotational symmetry More specifically, the heating parts 140 are disposed at four corners of the internal housing 101 (In **FIGS. 8** and **9****,** one of the heating parts 140 is hidden by the mounting parts 120 or the like and is not visible). Each of the heating parts 140 of this embodiment is formed in a substantially U-like shape and is a heater such as a fin heater or the like.

As illustrated in **FIG. 7****,** at the front side of the internal housing 101, there are provided opening/closing doors 105 which are installed in a corresponding relationship with the respective mounting parts 120 and which are used to mount the second airtight container 75 on each of the mounting parts 120 or to take out the second airtight container 75 from above each of the mounting parts 120. In this embodiment, nine opening/closing doors 105 are provided and are respectively positioned in a corresponding relationship with the respective mounting parts 120. The number of the mounting parts 120 and the number of the opening/closing doors 105 are equal to each other (nine in this embodiment).

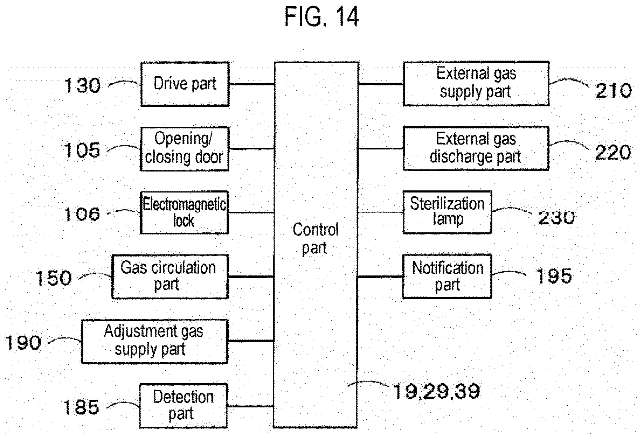

The aforementioned internal housing 101 includes four thermal insulation doors 101a. In this embodiment, there is used an aspect in which the opening/closing doors 105 are installed only in the thermal insulation door 101a disposed at the front side. However, the present disclosure is not limited thereto. The opening/closing doors 105 may be installed in a plurality of thermal insulation doors 101a (e.g., two thermal insulation doors 101a, three thermal insulation doors 101a or four thermal insulation doors 101a). By installing the opening/closing doors 105 in the plurality of thermal insulation doors 101a in this way, it is possible for the in-device transfer part 13, 23 or 33 to gain an access to the internal housing 101 from all directions. This makes it possible to increase the processing efficiency. Electromagnetic locks 106 *(see* **FIG. 14**) are installed in the opening/closing doors 105. When the carbon dioxide concentration within the internal housing 101 is high, when the oxygen concentration within the internal housing 101 is low, or when the below-mentioned high-temperature sterilization is performed, the control parts 19, 29 and 39 cause the electromagnetic locks 106 to surely lock the opening/closing doors 105. Thus, according to this embodiment, it is possible to realize high safety.

The four thermal insulation doors 101a of this embodiment can be removed *(see* **FIG**. **8**). Thus, for example, when the incubator part 100 is subjected to maintenance, it is possible to remove the thermal insulation doors 101a. This assures that the maintainability of the incubator part 100 becomes extremely superior. The thermal insulation doors 101 a are formed in a hybrid structure using two kinds of thermal insulation materials. One of the thermal insulation materials may be, for example, a vacuum insulation material.

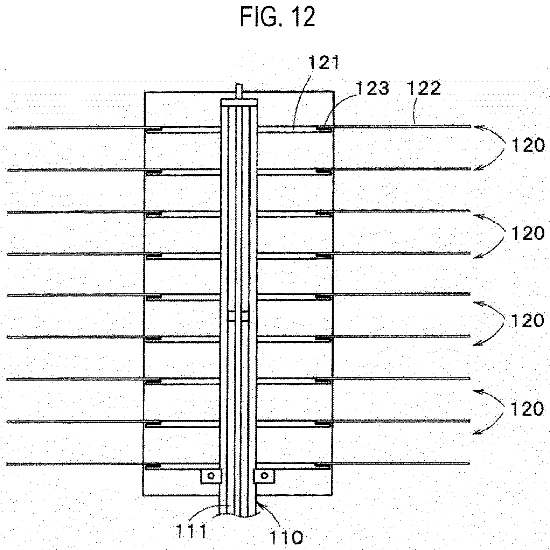

As illustrated in **FIG. 12****,** each of the mounting parts 120 of this embodiment includes: a disc-shaped first mounting portion 121 which is held by the central shaft 110 and opened at the center thereof; a disc-shaped second mounting portion 122 which is positioned outside a peripheral edge of the first mounting portion 121, held by the first mounting portion 121 through a below-mentioned elastic member 123 and opened at the center thereof; and the elastic member 123 interposed between the first mounting portion 121 and the second mounting portion 122.

Each of the mounting parts 120 is held by the central shaft 110 and is disposed above the adjoining lower mounting part 120. If the number of the mounting parts 120 does not exceed the upper limit that can be held by the central shaft 110, an arbitrary number of mounting parts 120 may be installed. Thus, according to this embodiment, the culture of cells can be performed by adjusting the number of the mounting parts 120 depending on the necessity.

As described above, each of the mounting parts 120 is merely disposed above the adjoining lower mounting part 120 and is not fixed to the adjoining lower mounting part 120. Each of the mounting parts 120 can be moved in the up-down direction with respect to the adjoining lower mounting part 120. In the meantime, the first mounting portion 121 of each of the mounting parts 120 is held by the central shaft 110. Therefore, each of the first mounting portions 121 cannot move in the horizontal direction.

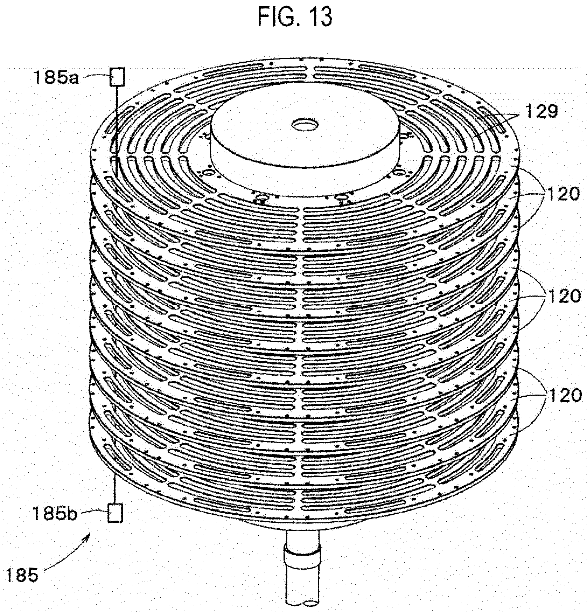

As described above, in this embodiment, there is provided a structure in which each of the mounting parts 120 is merely disposed above the respective adjoining lower mounting part 120. Extra wiring lines or pipelines are not used. Therefore, it is possible to culture cells while rotating the second airtight container 75 mounted on each of the mounting parts 120. Needless to say, the second airtight container 75 mounted on each of the mounting parts 120 may not be rotated and the drive part 130 may be stopped during the culture of cells. Furthermore, in accordance with the present embodiment, when culturing cells, as compared with a case where the below-described detection part 185 *(see* **FIG. 13**) determines whether the second airtight container 75 is mounted on each of the mounting parts 120, it is possible to rotate the mounting parts 120 at an extremely low speed. In this case, while rotating the mounting parts 120, the stress of vibration received by cells can be suppressed as far as possible.

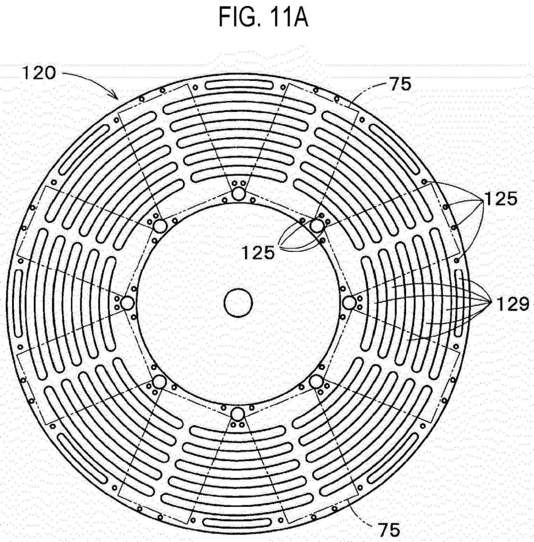

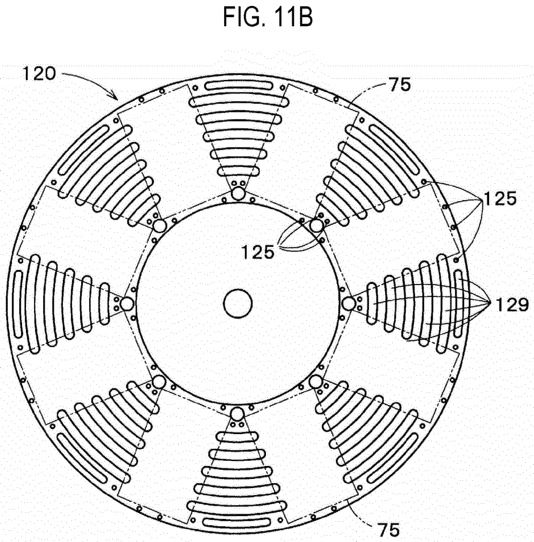

As illustrated in **FIGS. 11A** and **11B****,** each of the mounting parts 120 of this embodiment includes a disc-shaped plate having a plurality of arc-shaped slits formed in a concentric relationship. Furthermore, the arc-shaped slits adjoining in the circumferential direction are closed in an expected container mounting position to form a closed portion. As illustrated in **FIG. 11B****,** a distance of the closed portion in the circumferential direction may be substantially equal to a distance of the second airtight container 75, which is measured along the circumferential direction. By employing this aspect, an air flow can be kept substantially the same in the case where the second airtight container 75 is mounted on each of the mounting parts 120 and in the case where the second airtight container 75 is not mounted on each of the mounting parts 120. Even if the number of the second airtight containers 75 mounted on each of the mounting parts 120 is changed, it is possible to reduce the change of an air flow. In the case of employing the aspect illustrated in **FIG. 11B****,** the second airtight container 75 is mounted on each of the mounting parts 120 so that the film of the second airtight container 75 is positioned at the upper side.

In addition, each of the mounting parts 120 includes a positioning portion configured to perform the positioning of the second airtight container 75. In this embodiment, the positioning portion is formed as a plurality of projection portions 125. More specifically, the projection portions 125 are disposed so that each of four corners of the second airtight container 75 having a substantially rectangular shape is sandwiched by a pair of projection portions 125. As described above, in this embodiment, it is possible to mount the second airtight container 75 in a predetermined position. As a result, even in a state in which the second airtight containers 75 are mounted at the maximum number, it is possible to allow a gas to flow within the internal housing 101 as simulated in advance. This makes it possible to enhance the temperature stability and the heating response within the internal housing 101. In addition, in the aspect illustrated in **FIG. 11A****,** the second airtight container 75 may be mounted on each of the mounting parts 120 so that the film of the second airtight container 75 is positioned at the lower side. Alternatively, the second airtight container 75 may be mounted on each of the mounting parts 120 so that the film of the second airtight container 75 is positioned at the upper side.

As the drive part 130 illustrated in **FIG. 10****,** it is possible to employ, for example, a DD motor (direct drive motor) provided with an ABS ENC. (absolute encoder) therein. In this aspect, it is possible to accurately perform the positioning of the second airtight container 75 and to infinitely rotate the second airtight container 75 with low vibration. The present disclosure is not limited to this aspect. As the drive part 130, it may be possible to employ, for example, a stepping motor or an electric actuator which makes use of a timing belt.

As illustrated in **FIG. 10****,** the incubator part 100 includes a gas circulation part 150 which is installed in an upper central position within the internal housing 101 and is configured to draw a gas existing within the internal housing 101 from the lower side and to discharge the drawn gas in the circumferential direction. Outside a peripheral edge of the gas circulation part 150, there is disposed a diffusion member (diffuser) 160 which diffuses the gas discharged from the gas circulation part 150, within the internal housing 101 in a symmetrical relationship with respect to the axis of rotational symmetry *(see* **FIGS. 8** and **9**). In this embodiment, the diffusion member 160 is oriented toward the upper side of the respective heating parts 140. Thus, the diffusion member 160 is configured to diffuse the gas discharged in the circumferential direction from the gas circulation part 150 within the internal housing 101 so that the gas is blown toward the four corners of the internal housing 101 *(see* **FIG. 8**). The diffusion member 160 plays not only a function of applying a pressure to the diffused gas but also a function of a guide vane which guides the gas. The gas circulation part 150 of this embodiment is formed of, for example, a fan such as a sirocco fan, a centrifugal fan, an axial flow fan or the like.

In this embodiment, descriptions are made using an aspect in which the gas discharged from the gas circulation part 150 is blown toward the "upper side" of the respective heating parts 140. However, the present disclosure is not limited thereto. For example, the diffusion member 160 may be configured to "directly" blow the gas discharged from the gas circulation part 150 toward the respective heating parts 140.

As illustrated in **FIG. 8****,** the incubator part 100 further includes a heat insulating member 170 installed between the mounting parts 120 and the heating parts 140 so as to surround the peripheral edges of the mounting parts 120. A gap is formed at the lower side of the heat insulating member 170 between the heat insulating member 170 and a member adjoining the heat insulating member 170, namely the upper surface of "the bottom portion of the internal housing 101" in this embodiment.

As illustrated in **FIG. 8****,** a humidification water tray 180 (corresponding to a "humidification water storage part" of the claims) configured to store humidification water is mounted under the aforementioned mounting parts 120. An example of a material of the humidification water tray 180 may include a copper alloy stainless steel or a copper alloy. Employing this aspect is beneficial in that it is possible to sterilize the humidification water.

In this embodiment, the gas is discharged from the gas circulation part 150 (*see* **FIG. 10**). The gas discharged from the gas circulation part 150 in this way is guided by the diffusion member 160 and is blown toward the upper side of the respective heating parts 140 as indicated by a solid line arrow in **FIG. 8****.** Then, the gas blown toward the upper side of the respective heating parts 140 is heated by the heating parts 140 while mowing down along the outer periphery side of the heat insulating member 170. Then, the gas moving down to the upper surface of the bottom portion of the internal housing 101 passes through the gap between the heat insulating member 170 and the upper surface of the bottom portion of the internal housing 101 and flows into a region existing at the inner periphery side of the heat insulating member 170. The gas moves upward in the region and heats the mounting parts 120 and the second airtight containers 75 (*see* a dot line arrow in **FIG. 8**), ultimately heating the cells stored within the second airtight containers 75. Thereafter, the gas is drawn into the gas circulation part 150. The gas drawn into the gas circulation part 150 in this way is discharged again from the gas circulation part 150, consequently repeating the aforementioned flow.

The reason why the gas moves upward in the region existing at the inner periphery side of the heat insulating member 170 is because the gas is heated by the heating parts 140 and because the gas receives a suction force by which the gas is drawn into the gas circulation part 150. In **FIG. 8****,** there exists a region in which the heat insulating member 170 is not disposed. This region is located at the front side of the internal housing 101 and is positioned so as to face the inner surfaces of the opening/closing doors 105. Through the region in which the heat insulating member 170 is not disposed, the in-device transfer parts 13, 23 and 33 mount the second airtight containers 75 on the respective mounting parts 120 accommodated within the internal housing 101 or take out the second airtight containers 75 from above the respective mounting parts 120.

As illustrated in **FIG. 10****,** the incubator part 100 of this embodiment includes an adjustment gas supply part 190 for supplying an adjustment gas which is carbon dioxide and/or nitrogen, and an adjustment gas sterilization part (e.g., an ultraviolet sterilization lamp) 195 for sterilizing the adjustment gas supplied from the adjustment gas supply part 190 with ultraviolet rays or the like. By sterilizing the adjustment gas, which is carbon dioxide and/or nitrogen, with the adjustment gas sterilization part 195 in this way, it is possible to sterilize microorganisms or bacteria contained in the adjustment gas. Furthermore, a small amount of oxygen is contained in the adjustment gas. Therefore, in the case where an ultraviolet sterilization lamp is employed as the adjustment gas sterilization part 195, an ozone gas is generated from oxygen by the ultraviolet rays irradiated from the ultraviolet sterilization lamp. This makes it possible to always sterilize the interior of the incubator part 100 with the ozone gas.

A guide pipe 111 for guiding the adjustment gas supplied from the adjustment gas supply part 190 is disposed within the aforementioned central shaft 110. The adjustment gas passed through the guide pipe 111 reaches the lower side of the gas circulation part 150. The adjustment gas arrived at the lower side of the gas circulation part 150 in this way is drawn into the gas circulation part 150 form the lower side thereof and is discharged to the outside of the peripheral edge of the gas circulation part 150. The gas discharged from the gas circulation part 150 in this way is guided by the diffusion member 160 (*see* **FIGS. 8** and **9**) and is blown toward the upper side of the respective heating parts 140. The adjustment gas supply part 190 and the guide pipe 111 disposed within the central shaft 110 are connected to each other through a rotary joint 112.

As illustrated in **FIG. 13****,** the incubator part 100 of this embodiment includes a detection part 185 for detecting whether the second airtight container 75 is mounted on each of the mounting parts 120. The detection part 185 of this embodiment includes a light-emitting part 185a positioned higher than the mounting part 120 located at the uppermost side and configured to emit light downward, and a light-receiving part 185b positioned lower than the mounting part 120 located at the lowermost side and configured to receive the light emitted from the light-emitting part 185a. This aspect is nothing more than one example. As an alternative example, a light-emitting part may be positioned lower than the mounting part 120 located at the lowermost side and may be configured to emit light upward. A light-receiving part for receiving the light emitted from the light-emitting part may be positioned higher than the mounting part 120 located at the uppermost side. Opening portions 129 are formed in a portion of each of the mounting parts 120. The second airtight container 75 is mounted on at least a portion of each of the opening portions 129 *(see* **FIGS. 11A** and **11B**). Thus, when the light emitted from the light-emitting part 185a passes through the mounting position of the second airtight container 75, if the light-receiving part 185b receives the light with intensity equal to or larger than a threshold value, the detection part 185 can detect that the second airtight container 75 is not mounted in the mounting position of the second airtight container 75. On the other hand, if the light-receiving part 185b fails to receive the light or if the light-receiving part 185b receives the light with intensity smaller than a threshold value, the detection part 185 can detect that the second airtight container 75 is mounted in the mounting position of the second airtight container 75.

In this embodiment, the mounting parts 120 are held by the central shaft 110 so that the horizontal positions of the opening portions 129 of the respective mounting parts 120 are aligned with each other. Thus, by merely going around one of the mounting parts 120, it is possible to detect whether or not the second airtight containers 75 are mounted on all the mounting parts 120.

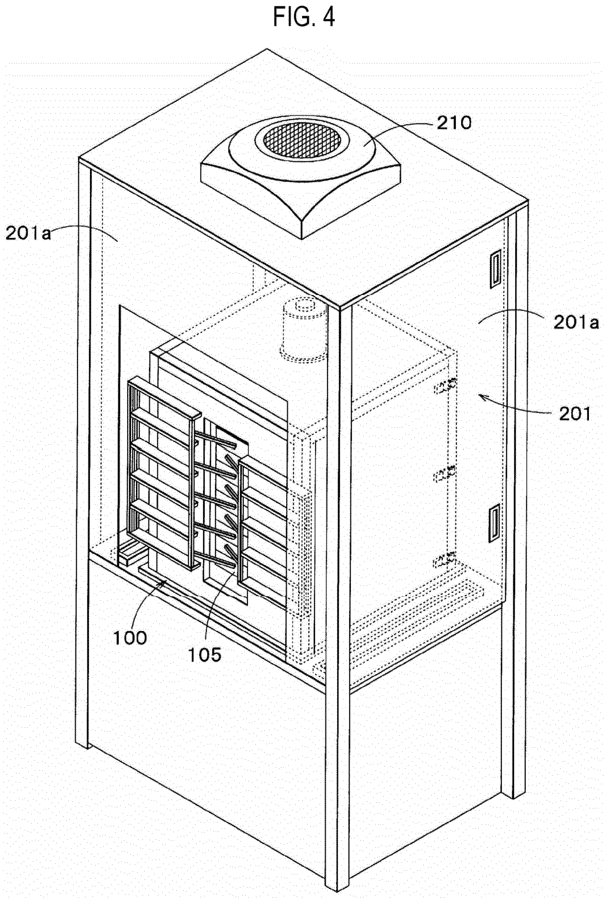

As illustrated in **FIG. 4****,** in this embodiment, an external housing 201 is installed so as to surround the internal housing 101 of the incubator part 100. The external housing 201 is opened at the front side of the internal housing 101 at which the opening/closing doors 105 are installed. Other devices including the in-device transfer parts 13, 23 and 33 are connected to the opened portion of the external housing 201.

The external housing 201 of this embodiment includes four maintenance doors 201 a. The respective maintenance doors 201a are removable. Thus, for example, when the incubator part 100 is subjected to maintenance, it is possible to remove the maintenance doors 201a. This assures that the maintainability of the incubator part 100 becomes extremely superior.

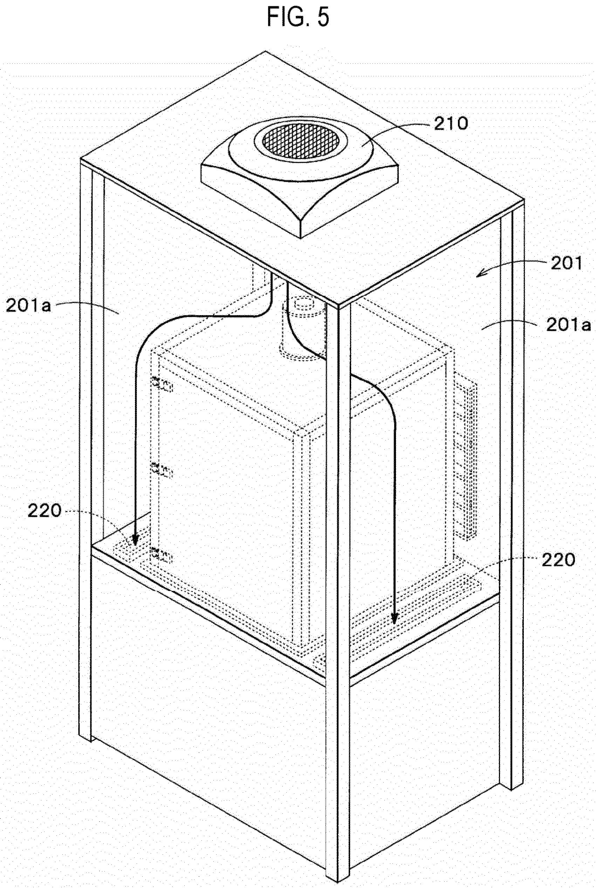

As illustrated in **FIG. 5****,** an external gas supply part 210 configured to supply a gas into the external housing 201 is installed at the upper side of the interior of the external housing 201. An external gas discharge part 220 configured to discharge the gas supplied from the external gas supply part 210 is installed at the lower side of the interior of the external housing 201. The external gas supply part 210 is, for example, a fan filter unit (FFU) which includes a fan and a high-performance filter (HEPA filter). By employing the fan filter unit (FFU), it is possible to keep the interior of the external housing 201 in a clean environment. Furthermore, in this embodiment, the external gas discharge part 220 is disposed at the opposite sides of the internal housing 101 so that the external gas discharge part 220 can discharge the gas existing within the external housing 201 in a balanced manner *(see* arrows in **FIG. 5**). The external gas discharge part 220 includes a louver (not shown). By adjusting an opening degree of the louver, it is possible to regulate a gas flow velocity or a gas pressure within the external housing 201.

As illustrated in **FIG. 6****,** a sterilization part formed of a sterilization lamp 230 for sterilizing the gas supplied from the external gas supply part 210 is installed within the external housing 201. The sterilization lamp 230 is disposed in the vicinity of a gas outlet 211 of the external gas supply part 210 and is configured to sterilize the gas blown out from the external gas supply part 210 by irradiating sterilization light such as ultraviolet light or the like on the gas. In this embodiment, there is used an aspect in which the sterilization part is formed of the sterilization lamp 230. However, this is nothing more than one example. It should be noted that other sterilization aspect may be used.

The maintenance doors 201 a described above is transparent or translucent. A material capable of preventing the sterilization light irradiated from the sterilization lamp 230 from leaking out of the external housing 201 is used as the material of the maintenance doors 201a. As an example, the material of the maintenance doors 201a may be polycarbonate which cuts ultraviolet light.

Furthermore, in this embodiment, as illustrated in **FIG. 6****,** a light shielding plate 240 for preventing the sterilization light irradiated from the sterilization lamp 230 from leaking out of the external housing 201 is installed under the sterilization lamp 230 at a predetermined inclination angle.

In the meantime, as illustrated in **FIG. 14****,** the control part 19, 29 and 39 of this embodiment are respectively connected to the drive part 130, the opening/closing doors 105, the electromagnetic locks 106, the gas circulation part 105, the adjustment gas supply part 190, the detection part 185, the external gas supply part 210, the external gas discharge part 220, the sterilization lamp 230 and the below-described notification part 195 so as to make communication therewith and are configured to control them.

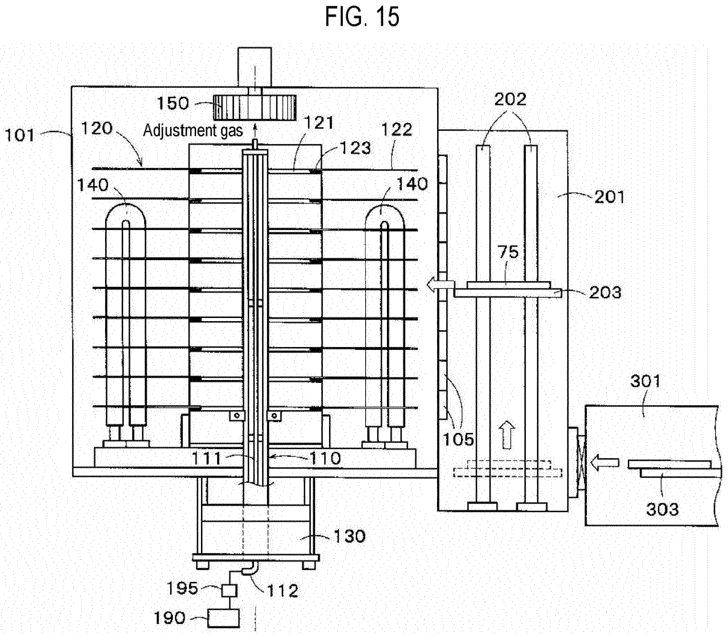

It is not necessarily essential that the external housing 201 is installed so as to surround the internal housing 101 of the incubator part 100 as illustrated in FIGS. 4 and 5. For example, as illustrated in FIG. 15, a delivery chamber 201 (corresponding to a "transfer housing" of the claims) disposed adjacent to the internal housing 101, a transfer mechanism 203 installed within the delivery chamber 201 and configured to transfer the second airtight container 75 into and out of the delivery chamber 201, and a up/down drive mechanism 202 configured to move the transfer mechanism 203 in the up-down direction, may be provided.

The delivery chamber 201 of this embodiment has a sealing property (air-tightness) with respect to the ambient air. The delivery chamber 201 is air-tightly connected to the internal housing 101 at the front side of the internal housing 101 at which the opening/closing doors 105 are installed. A transfer chamber 301 including the in-device transfer part 13, 23 or 33 and having a sealing property with respect to the ambient air is air-tightly connected to the delivery chamber 201. In **FIG. 15****,** the in-device transfer part 13, 23 or 33 is designated by reference numeral 303.

As indicated by arrows in **FIG. 15****,** in the case of loading the second airtight container 75 into the incubator part 100, the second airtight container 75 is carried from the transfer chamber 301 into the delivery chamber 201 by the in-device transfer part 303 and is mounted on the transfer mechanism 203. Subsequently, the second airtight container 75 is moved in the up-down direction together with the transfer mechanism 203 by the up/down drive mechanism 202 and is stopped in a height position corresponding to a target opening/closing door 105. After the target opening/closing door 105 is opened, the second airtight container 75 is carried from the transfer chamber 301 into the internal housing 101 by the transfer mechanism 203 and is mounted on the mounting part 120. In the case of unloading the second airtight container 75 from the incubator part 100, the aforementioned process is performed in the reverse order. Thus, the second airtight container 75 is taken out from the mounting part 120 to the in-device transfer part 303.

In some embodiments, a heating part (not shown) may be installed in the delivery chamber 201. An internal atmosphere of the delivery chamber 201 is controlled so that the temperature becomes, for example, 37 degrees C. For example, a heater such as a fin heater or the like may be used as the heating part.

Furthermore, in some embodiments, an H₂O₂ gas supply part (not shown) may be connected to the delivery chamber 201 so that the H₂O₂ gas supply part can periodically sterilize the interior of the delivery chamber 201 using an H₂O₂ gas. In this case, it is possible to prevent the interior of the internal housing 101 of the incubator part 100 from being contaminated by the internal atmosphere of the delivery chamber 201.

### «Effects»

Next, descriptions will be made on the effects not yet mentioned or the especially important effects among the effects achieved by the embodiments having the aforementioned configuration.

According to this embodiment, as illustrated in **FIGS. 8** and **9****,** the heating parts 140 are disposed in a symmetrical relationship with respect to the axis of rotational symmetry *(see* **FIG. 10**) extending in the up-down direction. The diffusion member 160 diffuses the gas discharged from the gas circulation part 150 within the internal housing 101 in a symmetrical relationship with respect to the axis of rotational symmetry. Thus, it is possible to make uniform, with high accuracy, the temperature, the gas distribution or the like within the internal housing 101 of the incubator part 100. As a result, it is possible to stably culture the cells existing within the second airtight container 75.

Furthermore, in this embodiment, the diffusion member 160 diffuses the gas discharged from the gas circulation part 150 within the internal housing 101 so that the gas is blown toward the upper side of the respective heating parts 140. Thus, the gas discharged from the gas circulation part 150 can be instantly heated by the heating parts 140 to create an ascending gas flow. This makes it possible to activate a gas flow within the internal housing 101. Accordingly, it is possible to make uniform, with high accuracy, the temperature, the gas distribution or the like within the internal housing 101. By instantly creating the ascending gas flow in this way, it is possible to make active the up-down movement of a gas within the internal housing 101. It can be expected that when the opening/closing doors 105 are opened, the ascending gas flow functions as a so-call air curtain. As a result, when the opening/closing doors 105 are opened, it is possible to prevent the temperature from being sharply reduced and to prevent the gas concentration from being abruptly changed. In addition, the aforementioned effects can be achieved even when employing an aspect in which the diffusion member 160 blows a gas toward the respective heating parts 140.

In this embodiment, as illustrated in **FIG. 8****,** the heat insulating member 170 is installed between the mounting parts 120 and the heating parts 140 so as to surround the peripheral edges of the mounting parts 120. Thus, it is possible to prevent the second airtight container 75, ultimately the cells existing within the second airtight container 75, from being directly heated by the heating parts 140. Thus, in this embodiment, it is possible to reliably culture the cells at a uniform temperature.

As the heat insulating member 170, it may be possible to employ a heat insulating member, at least the inner surface of which is formed of a mirror surface made of, for example, stainless steel. In the case of employing this aspect, it is possible to reflect the heat coming from the inner periphery of the heat insulating member 170 toward the inner periphery and to efficiently heat the second airtight container 75, ultimately the cells existing within the second airtight container 75. In addition, in the case where the heating parts 140 are formed of infrared heaters which emit infrared rays, the entirety of the heat insulating member 170 is heated by infrared rays. The infrared rays existing at the inner periphery side of the heat insulating member 170 are reflected by the mirror surface of the heat insulating member 170. This makes it possible to efficiently heat the cells existing within the second airtight container 75.

In view of the maintenance of the incubator part 100, the heat insulating member 170 may be detachably installed in the internal housing 101 (*see* **FIG. 9**). In **FIG. 9****,** there is illustrated an aspect in which the humidification water tray 180 is also removed.

Furthermore, in this embodiment, it is possible to culture the cells while rotating the second airtight container 75 on each of the mounting parts 120 with the drive part 130. Thus, even if a bias in the temperature, the gas distribution, the humidity or the like is generated within the internal housing 101 for some reason, it is possible to culture the cells under uniform conditions.

As illustrated in **FIG. 10****,** the incubator part 100 of this embodiment includes the adjustment gas supply part 190 for supplying an adjustment gas which is nitrogen and/or carbon dioxide. Thus, in this embodiment, it is possible to appropriately adjust the concentration of the adjustment gas in the gas existing within the internal housing 101.

Furthermore, in this embodiment, the adjustment gas supplied from the adjustment gas supply part 190 reaches the lower side of the gas circulation part 150. The adjustment gas is drawn into the gas circulation part 150 from the lower side thereof and is discharged toward the outside of the peripheral edge of the gas circulation part 150. The gas discharged from the gas circulation part 150 in this way is guided by the diffusion member 160 and is blown toward the upper side of the respective heating parts 140. Thus, in this embodiment, even when the adjustment gas is newly supplied, it is possible to immediately draw the adjustment gas with the gas circulation part 150 and to diffuse the adjustment gas into the internal housing 101. It is therefore possible to make uniform the gas concentration in the internal housing 101 within an extremely short period of time.

The opening/closing doors 105 illustrated in **FIG. 7** are installed in a corresponding relationship with the respective mounting parts 120. The second airtight container 75 can be mounted on each of the mounting parts 120 through each of the opening/closing doors 105 or can be taken out from above each of the mounting parts 120 through each of the opening/closing doors 105. Thus, when the in-device transfer part 13, 23 or 33 gains an access to the interior of the incubator part 100, it is possible to open only the necessary one of the opening/closing doors 105. This makes it possible to restrain, as far as possible, the temperature, a gas component, the humidity or the like within the internal housing 101 from being changed when the in-device transfer part 13, 23 or 33 gains an access to the interior of the incubator part 100. Furthermore, by employing this aspect, the second airtight container 75 can be mounted on each of the mounting parts 120 or can be taken out from above each of the mounting parts 120 without having to move the mounting parts 120 up and down. Thus, as compared with an aspect where the mounting parts 120 are moved up and down, it is possible to reduce the stress applied to the cells under culture and to prevent the internal atmosphere of the internal housing 101 from being disturbed.

In general, there is a need to periodically sterilize the incubator part 100 at a high temperature. However, when the incubator part 100 is sterilized at a high temperature in this way, the internal temperature of the incubator part 100 becomes close to, for example, 180 degrees C. If the second airtight container 75 is mounted on of the mounting part 120 during the high temperature sterilization, the cells cultured within the second airtight container 75 may be killed or adversely affected. In view of this, the incubator part 100 of this embodiment includes the detection part 185 for detecting whether the second airtight container 75 is mounted on the mounting part 120 (*see* **FIG. 13**). Thus, it is possible to prevent the incubator part 100 from being sterilized at a high temperature in a state in which the second airtight container 75 is mounted on the mounting part 120. When performing the high temperature sterilization in this way, the detection part 185 may automatically detect whether the second airtight container 75 is mounted on the mounting part 120. More specifically, prior to performing the high temperature sterilization, the mounting part 120 may be necessarily rotated by the drive part 130 while allowing the light-emitting part 185a to emit light, so as to detect whether the second airtight container 75 is mounted on the mounting part 120. If it is determined by the detection part 185 that the second airtight container 75 is mounted on the mounting part 120, this situation may be notified using the notification part 195 *(see* **FIG. 14**), the external device 90 *(see* **FIG**. **2**) or the like, or the inability to perform the high temperature sterilization may be notified using the notification part 195, the external device 90 or the like.

As described above, when the incubator part 100 is subjected to the high temperature sterilization, the internal temperature of the incubator part 100 becomes close to 180 degrees C. In view of this, in this embodiment, as illustrated in **FIG. 12****,** the elastic member 123 is disposed between the first mounting portion 121 and the second mounting portion 122. Furthermore, each of the mounting parts 120 is merely mounted above the adjoining lower mounting part 120 and can be moved in the up-down direction with respect to the adjoining lower mounting part 120. Thus, even if the mounting parts 120 or the central shaft 110 is largely expanded under the influence of heat during the high temperature sterilization, it is possible to permit the expansion in both the up-down direction and the horizontal direction. As a result, it is possible to prevent the mounting parts 120 or the central shaft 110 from being distorted by heat expansion.

In some embodiments, instead of sterilizing the incubator part 100 at a high temperature, it may be possible to periodically sterilize the incubator part 100 using an H₂O₂ gas. Similar to the high temperature sterilization, the sterilization of the incubator part 100 using the H₂O₂ gas is performed in a state in which the second airtight container 75 is not mounted on the mounting part 120.

In this embodiment, as illustrated in **FIG. 8****,** the humidification water tray 180 which stores humidification water is disposed under the mounting parts 120. Thus, it is possible to keep the interior of the internal housing 101 at high humidity. In the case where the second airtight container 75 is used as mainly described thus far, there is no need to keep the interior of the internal housing 101 at high humidity. However, in the case where a culture-purpose dish, a culture-purpose petri dish rather than the second airtight container 75 is used as the "container", it is of important significance to keep the interior of the internal housing 101 at high humidity as mentioned above.

In the example illustrated in **FIGS. 4** to **6****,** the external housing 201 is disposed so as to surround the internal housing 101. Furthermore, the external gas supply part 210 configured to supply a gas into the external housing 201 is installed at the upper side of the interior of the external housing 201. Moreover, the external gas discharge part 220 configured to discharge the gas supplied from the external gas supply part 210 is installed at the lower side of the interior of the external housing 201. Thus, it is possible to dispose the incubator part 100 in a clean environment and to reduce a contamination risk. In the case where a fan filter unit is employed as the external gas supply part 210, it is possible to dispose the incubator part 100 in a cleaner environment and to further reduce a contamination risk.

Furthermore, in the example illustrated in **FIGS. 4** to **6****,** there is installed the sterilization part which includes the sterilization lamp 230 for sterilizing the gas supplied from the external gas supply part 210 (*see* **FIG. 6**). Thus, the gas sterilized with the sterilization lamp 230 can be supplied from the upper side of the incubator part 100. Accordingly, it is possible to dispose the incubator part 100 in a cleaner environment and to further reduce a contamination risk.

Moreover, in the example illustrated in **FIGS. 4** to **6****,** the light shielding plate 240 for preventing the sterilization light irradiated from the sterilization lamp 230 from leaking out of the external housing 201 is installed under the sterilization lamp 230. Thus, it is possible to prevent the sterilization light irradiated from the sterilization lamp 230 from leaking to the outside and affecting the second airtight container 75 or the like.

In addition, in the example illustrated in **FIGS. 4** to **6****,** the maintenance doors 201 a are made of polycarbonate or the like and are formed of a material which prevents the sterilization light irradiated from the sterilization lamp 230 from leaking out of the external housing 201. Thus, it is possible to reliably prevent the sterilization light such as ultraviolet light or the like irradiated from the sterilization lamp 230 from leaking the outside and affecting a human body or the like.

In the example illustrated in **FIG. 15****,** the delivery chamber 201 disposed adjacent to the internal housing 101, the transfer mechanism 203 installed within the delivery chamber 201 and configured to move the second airtight container 75 into and out of the delivery chamber 201, and the up/down drive mechanism 202 configured to move the transfer mechanism 203 in the up-down direction, are provided. Since the transfer chamber 301 including the in-device transfer part 303 is not directly connected to the internal housing 101 of the incubator part 100 but is connected the internal housing 101 via the delivery chamber 201, it is possible to further reduce a change in the internal environment of the internal housing 101 and to prevent introduction (contamination) of bacteria from the outside. In addition, since the up/down drive mechanism 202 is installed in the delivery chamber 201, it is not necessary to install an up/down drive mechanism in the in-device transfer part 303. This makes it possible to simplify the mechanism (configuration) of the in-device transfer part 303.

### «Example»

An example of the incubator part 100 of the culturing device according to this embodiment will be described.

The temperature performance was measured using the incubator part 100 of the culturing device according to this embodiment. Over a time period of 30 minutes after the internal temperature of the internal housing 101 is stabilized, the fluctuation in the temperature was measured at one-minute intervals. When setting the internal temperature of the internal housing 101 at 37.0 degrees C, in the incubator part 100 according to this embodiment, the temperature fluctuation range was 37.0 ± 0.02 degrees C, the temperature distribution was 37.0 ± 0.2 degrees C, and the spatial temperature deviation was 0.20 degrees C. When carrying out the same experiment with respect to a commercially available incubator that does not employ the configuration of this embodiment, the temperature fluctuation range was 37.45 ± 0.05 degrees C, the temperature distribution was 37.45 ± 0.32 degrees C, and the spatial temperature deviation was 0.54 degrees C. In this way, according to the incubator part 100 of this embodiment, it was possible to realize extremely high temperature performance. Incidentally, in the experiment described above, the test standard of the temperature fluctuation range was in reference to JTM K01/03/05, the test standard of the temperature distribution was in reference to JTM K01/03/05, and the test standard of the spatial temperature deviation was in reference to JTM K07/09.

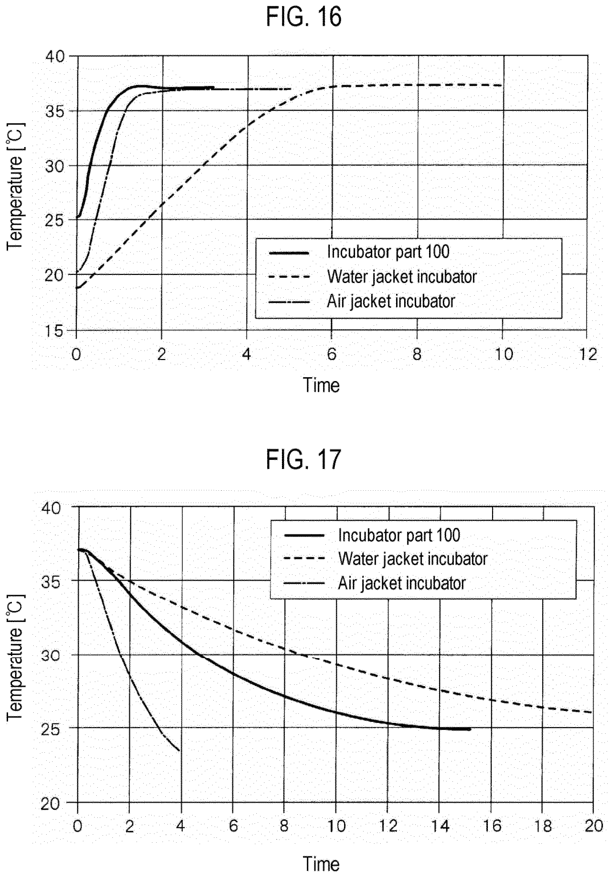

Furthermore, the temperature pull-up characteristics were measured using the incubator part 100 of the culturing device according to this embodiment. The results are shown in **FIG. 16****.** The solid line indicates the measurement result obtained by the incubator part 100 of this embodiment. The dotted line indicates the measurement result obtained by a commercially available water jacket incubator. The one-dot chain line indicates the measurement result obtained by a commercially available air jacket incubator. It can be understood that according to the incubator part 100 of this embodiment, the temperature rise is fast and the time taken until the temperature is stabilized is short. In addition, it can be understood that since the time taken until the temperature is stabilized is short, the temperature followability to the disturbance is high.

Furthermore, the temperature drop characteristics at the time of power outage were measured using the incubator part 100 of the culturing device according to this embodiment. The results are shown in **FIG. 17****.** The solid line indicates the measurement result obtained by the incubator part 100 of this embodiment. The dotted line indicates the measurement result obtained by a commercially available water jacket incubator. The one-dot chain line indicates the measurement result obtained by a commercially available air jacket incubator. It can be understood that the incubator part 100 of this embodiment exhibits thermal insulation performance much higher than that of the air jacket incubator and further that the incubator part 100 of this embodiment exhibits thermal insulation performance nearly unchanged from that of the water jacket incubator.

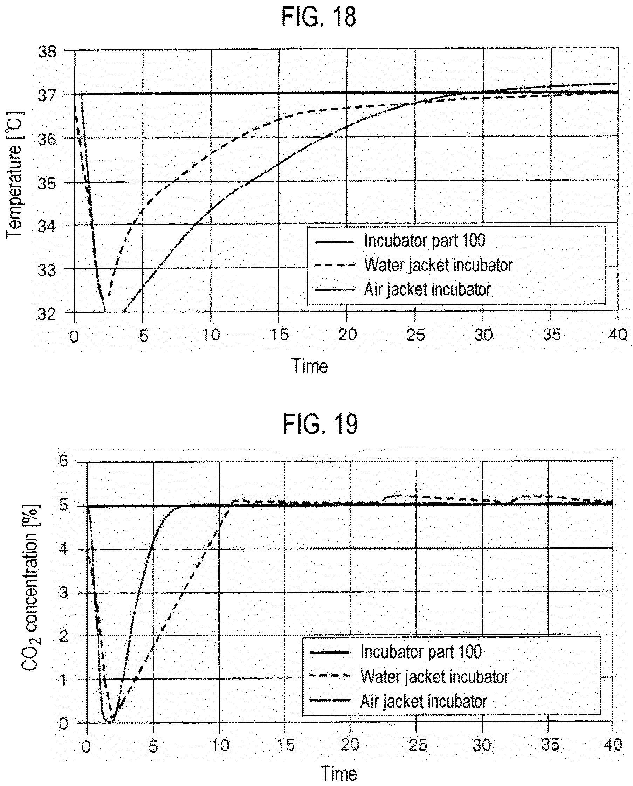

Furthermore, the temperature return characteristics at the time of door opening were measured using the incubator part 100 of the culturing device according to this embodiment. The door opening time period is 60 seconds. The results are shown in **FIG. 18****.** The solid line indicates the measurement result obtained by the incubator part 100 of this embodiment. The dotted line indicates the measurement result obtained by a commercially available water jacket incubator. The one-dot chain line indicates the measurement result obtained by a commercially available air jacket incubator. As shown in **FIG. 18****,** it can be understood that although the temperature was sharply dropped in the water jacket incubator and the air jacket incubator when doors are opened, the temperature was not affected in the incubator part 100 of this embodiment even when the opening/closing doors 105 are opened for a long period of time of 60 seconds. In this way, according to this embodiment, even when loading or unloading the airtight container, only a necessary minimum opening leads to the outside. This makes it possible to eliminate an abrupt change in temperature. It is therefore possible to reduce a thermal stress applied to the cells under culture as far as possible. Furthermore, according to this embodiment, the temperature is not dropped even when the opening/closing doors 105 are opened for a long period of time of 60 seconds. It is therefore possible to achieve energy saving. Moreover, it can be understood that the incubator part 100 of this embodiment remains strong against disturbance. The time period of 60 seconds mentioned above corresponds to, for example, the time period required in loading and unloading the second airtight container 75 transferred to the next step.

Furthermore, the carbon dioxide concentration return characteristics at the time of door opening were measured using the incubator part 100 of the culturing device according to this embodiment. The door opening time period is 60 seconds. The results are shown in **FIG. 19****.** The solid line indicates the measurement result obtained by the incubator part 100 of this embodiment. The dotted line indicates the measurement result obtained by a commercially available water jacket incubator. The one-dot chain line indicates the measurement result obtained by a commercially available air jacket incubator. As shown in **FIG. 19****,** it can be understood that although the carbon dioxide concentration was sharply dropped in the water jacket incubator and the air jacket incubator when doors are opened, the carbon dioxide concentration was not affected in the incubator part 100 of this embodiment even when the opening/closing doors 105 are opened for a long period of time of 60 seconds. In this way, the carbon dioxide concentration is not reduced even when the opening/closing doors 105 are opened for a long period of time. Thus, according to the incubator part 100 of this embodiment, it is possible to achieve energy saving. Moreover, it can be understood that the incubator part 100 of this embodiment remains strong against disturbance.

Finally, the foregoing descriptions of the embodiment and the disclosure of the drawings are nothing more than one example for describing the present disclosure recited in the claims. The present disclosure recited in the claims shall not be limited by the foregoing descriptions of the embodiment and the disclosure of the drawings.

## Claims

1. A culturing device, comprising:
an incubator part,
wherein the incubator part includes:
an internal housing;
a mounting part installed within the internal housing, and configured to mount a container which stores cells;
a plurality of heating parts installed within the internal housing, and disposed in a symmetrical relationship with respect to an axis of rotational symmetry extending in an up-down direction;
a gas circulation part installed in an upper central position within the internal housing, and configured to draw a gas existing within the internal housing and to discharge the gas to be drawn from the internal housing; and
a diffusion member configured to diffuse the gas discharged by the gas circulation part within the internal housing in a symmetrical relationship with respect to the axis of rotational symmetry.

2. The culturing device of Claim 1, wherein the diffusion member is configured to diffuse the gas discharged by the gas circulation part within the internal housing so that the gas is blown toward each of the heating parts or an upper side of each of the heating parts.

3. The culturing device of Claim 1 or 2, wherein the incubator part further includes: a heat insulating member installed between the mounting part and the heating parts so as to surround at least a portion of a peripheral edge of the mounting part, and
a gap is formed at a lower side of the heat insulating member between the heat insulating member and a member adjoining the heat insulating member.

4. The culturing device of any one of Claims 1 to 3, wherein the incubator part further includes: a drive part configured to rotate the mounting part about the axis of rotational symmetry.

5. The culturing device of any one of Claims 1 to 4, wherein the incubator part further includes an adjustment gas supply part configured to supply an adjustment gas to the gas circulation part, and
the adjustment gas supply part adjusts a concentration of the adjustment gas in the gas existing within the internal housing.

6. The culturing device of Claim 5, further comprising:
an adjustment gas sterilization part configured to sterilize the adjustment gas supplied from the adjustment gas supply part.

7. The culturing device of Claim 6, wherein the adjustment gas includes oxygen, and the adjustment gas sterilization part is configured to irradiate ultraviolet rays onto the adjustment gas.

8. The culturing device of any one of Claims 1 to 7, wherein the incubator part further includes a humidification water storage part disposed under the mounting part and configured to store humidification water.

9. The culturing device of any one of Claims 1 to 8, wherein the incubator part further includes a detection part configured to detect whether the container is mounted on the mounting part.

10. The culturing device of any one of Claims 1 to 9, wherein the mounting part includes a plurality of mounting parts, and
the internal housing includes opening/closing doors installed in a corresponding relationship with the respective mounting parts.

11. The culturing device of Claim 10, wherein the number of the mounting parts is equal to the number of the opening/closing doors.

12. The culturing device of any one of Claims 1 to 11, wherein the incubator part further includes a holding part extending in the up-down direction along the axis of rotational symmetry and configured to hold the mounting part in a horizontal direction, and
the mounting part includes a first mounting portion held by the holding part, a second mounting portion positioned outside a peripheral edge of the first mounting portion and held by the first mounting portion, and an elastic member disposed between the first mounting portion and the second mounting portion.

13. The culturing device of any one of Claims 1 to 12, wherein the mounting part includes a plurality of mounting parts, and
each of the plurality of mounting parts is mounted above an adjoining lower mounting part so that each of the plurality of mounting parts can be moved in the up-down direction with respect to the adjoining lower mounting part.

14. The culturing device of any one of Claims 1 to 13, further comprising:
an external housing disposed so as to surround the internal housing;
an external gas supply part installed at an upper side within the external housing and configured to supply a gas into the external housing; and
an external gas discharge part installed at a lower side within the external housing and configured to discharge the gas supplied from the external gas supply part.

15. The culturing device of any one of Claims 1 to 14, further comprising:
an external housing disposed so as to surround the internal housing; and
a sterilization part installed within the external housing and configured to sterilize the gas supplied from the supply part.

16. The culturing device of Claim 15, wherein the sterilization part is a sterilization lamp, and
a light shielding plate configured to prevent sterilization light irradiated from the sterilization lamp from leaking out of the external housing is installed below the sterilization lamp.

17. The culturing device of Claim 15 or 16, wherein the sterilization part is a sterilization lamp,
at least a portion of the external housing is transparent or translucent, and
a transparent or translucent portion of the external housing is made of a material which prevents sterilization light irradiated from the sterilization lamp from leaking out of the external housing.

18. The culturing device of any one of Claims 1 to 13, further comprising:
a delivery housing disposed adjacent to the internal housing;
a transfer mechanism installed within the delivery housing and configured to transfer the container into and out of the internal housing; and
an up/down drive mechanism configured to move the transfer mechanism in the up-down direction.

19. The culturing device of any one of Claims 1 to 18, wherein the mounting part includes a disc-shaped plate in which a plurality of arc-shaped slits is formed in a concentric relationship, and
the arc-shaped slits adjoining in the circumferential direction are closed in an expected container mounting position to form a closed portion.

20. The culturing device of Claims 19, wherein a distance of the closed portion in the circumferential direction is substantially equal to a distance of the container in the circumferential direction.
